# EUROPEAN PATENT SPECIFICATION

(11) **EP 4 319 643 B1**
(45) Date of publication and mention of the grant of the patent: **25.11.2026**
(21) Application number: 22721230.5
(22) Date of filing: 15.04.2022
(51) Int. Cl.: A61B 8/08, A61B 8/00

(54) **A MEDICAL DEVICE SYSTEM HAVING BLOOD VESSEL CORRELATION TOOLS**
EIN MEDIZINPRODUKTSYSTEM MIT BLUTGEFÄSS-KORRELATIONSWERKZEUGEN
SYSTÈME DE DISPOSITIF MÉDICAL COMPORTANT DES OUTILS DE CORRÉLATION DES VAISSEAUX SANGUINS

(30) Priority: 15.04.2021 US 202163175484 P
(43) Date of publication of application: 14.02.2024
(73) Proprietor: Bard Access Systems, Inc., Salt Lake City, UT 84116 (US)
(72) Inventor: SOWARDS, Steffan, Salt Lake City, UT 84124 (US); MCLAUGHLIN, William, Robert, Bountiful, UT 84010 (US); MISENER, Anthony, K., Bountiful, UT 84010 (US)
(74) Representative: Hoffmann Eitle
(86) International application number: PCT/US2022/025097
(87) International publication number: WO 2022/221714

(56) References cited:
- EP-A1- 2 823 766
- WO-A1-2016/006769
- US-A1- 2013 197 367
- US-A9- 2005 119 546

## Description

### PRIORITY

This application claims the benefit of priority to U.S. Provisional Application No. 63/175,484, filed April 15, 2021.

### BACKGROUND

Readily detecting and accessing a blood vessel can require a user to detect the blood vessel in one location and confirm different parameters of the detected blood vessel in another distinct location. Clinicians may use an ultrasound probe to detect one or more blood vessels within a target area but have to confirm different parameters of the blood vessels on a screen, breaking the clinician's focus from the target area to the screen. It would be beneficial for clinicians to have access to blood vessel visualization or blood vessel detection data close to or within the target area, allowing the clinicians to focus their full attention on the target area. Disclosed herein are a medical device system having blood vessel correlation tools and method of use that address the foregoing.

EP 2823766 A1 discloses an ultrasound system and method for determining an object, for example, an artery or a vein, and provide object information corresponding to a position of the objection. The ultrasound system includes: an ultrasound probe that transmits an ultrasound signal to a body comprising an object, and receives an ultrasound echo signal reflected from the body to generate a reception signal, an ultrasound data acquiring unit that acquires ultrasound data corresponding to the object in the body by using the reception signal, a processing unit that generates Doppler data by using the ultrasound data, analyzes the Doppler data to detect the object, and generates object information corresponding to a position of the detected object, and an object information providing unit that outputs the object information.

US 2013197367 A1 discloses a method and apparatus for locating and distinguishing blood vessel.

Further relevant prior art may be found in the patent publications US2005119546 A9 and WO 2016/006769 A1.

### SUMMARY

In a first aspect of the present invention, there is provided a medical device system according to claim 1.

In a second aspect of the present invention, there is provided a method according to claim 11.

The dependent claims define preferred embodiments.

Disclosed herein is a medical device system configured to detect one or more blood vessels within a target area. The medical device system includes an ultrasound probe configured to detect one or more blood vessels, the ultrasound probe in communication with a console. The medical device system further includes one or more blood vessel correlation tools configured to generate a local output detectable by a user. The one or more blood vessel correlation tools are coupled to the ultrasound probe and in communication with a console. The one or more blood vessel correlation tools are selected from the group consisting of a light array, one or more visible light projectors, a haptic feedback system, and an auditory device.

In some embodiments, the ultrasound probe is configured to detect the one or more blood vessels within the target area using ultrasound or near-infrared reflection differentiation.

In some embodiments, the console includes one or more processors, an energy source, non-transitory computer readable medium and a plurality of logic modules.

In some embodiments, the plurality of logic modules, when activated by the processor, are configured to perform operations including receiving detected blood vessel data from the ultrasound probe, correlating the detected blood vessel data with the local output corresponding to one or more parameters of the detected blood vessels, and generating the local output that is detected by a user.

In some embodiments, the one or more parameters include blood vessel location within the target area, blood vessel size, blood vessel type, blood vessel depth or vascular access devices that are configured to access the one or more blood vessels.

In some embodiments, the local output includes visible illumination of one or more lights in the light array, and the visible illumination of the one or more lights in the light array includes visible illumination of a different color, a different pattern, or of varied brightness corresponding to the one or more parameters of the detected blood vessels.

In some embodiments, the local output includes one or more visible light projections by the one or more visible light projectors on the target area.

In some embodiments, the one or more visible light projections display a blood vessel visualization depiction.

In some embodiments, the one or more visible light projections display different colors, different shapes, or varied brightness corresponding to the one or more parameters of the detected blood vessels.

In some embodiments, the local output includes an auditory signal from the auditory device.

In some embodiments, the auditory device emits sonic tones of various frequencies, various patterns, or various pitches corresponding to the one or more parameters of the detected blood vessels.

In some embodiments, the local output includes vibrations of a portion of the ultrasound probe through the haptic feedback system.

In some embodiments, the haptic feedback system vibrates in various patterns, various frequencies or various magnitudes corresponding to the one or more parameters of the detected blood vessels.

Also disclosed herein is a method of detecting one or more blood vessels within a target area and generating a local output detected by a user. The method includes detecting one or more blood vessels within a target area using an ultrasound probe, correlating detected blood vessel data with a local output corresponding to one or more parameters of the detected blood vessels, and generating the local output configured to be detected by a user using one or more blood vessel correlation tools.

In some embodiments, detecting one or more blood vessels in a target area includes using an ultrasound probe configured to detect the one or more blood vessels by ultrasound waves or near-infrared reflection differentiation.

In some embodiments, correlating detected blood vessel data with a local output corresponding to one or more parameters of the detected blood vessels includes the one or more parameters including blood vessel location, blood vessel type, blood vessel size, and vascular access devices configured to access the detected blood vessels.

In some embodiments, generating the local output configured to be detected by a user using one or more blood vessel correlation tools includes the one or more blood vessel correlation tools including a light array, one or more visible light projectors, a haptic feedback system, or an auditory device.

In some embodiments, the local output includes one or more lights in the light array being illuminated a different color, a different pattern, or a varied brightness, each of the different colors, patterns, or brightness corresponding to one parameter of the one or more parameters.

In some embodiments, the local output includes one or more blood vessel visualization depictions projected on the target area by the one or more visible light projectors, the one or more blood vessel visualization depictions including different colors or different shapes, each of the different colors or shapes corresponding to one parameter of the one or more parameters.

In some embodiments, the local output includes the haptic feedback system vibrating a portion of the ultrasound probe in various patterns, various frequencies, or various magnitudes, each of the various patterns, frequencies, or magnitudes corresponding to one parameter of the one or more parameters.

In some embodiments, the local output includes the auditory device emitting one or more sonic tones having various frequencies, pitches, or patterns, each of the various frequencies, pitches or patterns corresponding to one parameter of the one or more parameters.

These and other features of the concepts provided herein will become more apparent to those of skill in the art in view of the accompanying drawings and following description, which describe particular embodiments of such concepts in greater detail.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 illustrates a perspective view of a medical device system including an ultrasound probe having one or more ultrasound vessel correlation tools, in accordance with some embodiments.
FIG. 2 illustrates a block diagram of some components of the medical device system including the ultrasound probe and a console, in accordance with some embodiments.
FIG. 3A illustrates a perspective view of the ultrasound probe including a light array, in accordance with some embodiments.
FIG. 3B illustrates a perspective view of the ultrasound probe including one or more visible light projectors, in accordance with some embodiments.
FIG. 3C illustrates a perspective view of the ultrasound probe including a haptic feedback system, in accordance with some embodiments.
FIG. 3D illustrates a perspective view of the ultrasound probe including an auditory device, in accordance with some embodiments.
FIGS. 4A-4C illustrate a cross sectional view of an exemplary method of detecting one or more blood vessels within a target area and generating a local output configured to be detected by a user, in accordance with some embodiments.
FIG. 5 illustrates a flow chart of the exemplary method of detecting one or more blood vessels within a target area and correlating the detected blood vessels with a local output detected by a user, in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, an ultrasound probe disclosed herein includes a portion of the ultrasound probe intended to be near a clinician when the ultrasound probe is used on a patient. Likewise, a "proximal length" of, for example, the ultrasound probe includes a length of the ultrasound probe intended to be near the clinician when the ultrasound probe is used on the patient. A "proximal end" of, for example, the ultrasound probe includes an end of the ultrasound probe intended to be near the clinician when the ultrasound probe is used on the patient. The proximal portion, the proximal-end portion, or the proximal length of the ultrasound probe can include the proximal end of the ultrasound probe; however, the proximal portion, the proximal-end portion, or the proximal length of the ultrasound probe need not include the proximal end of the ultrasound probe. That is, unless context suggests otherwise, the proximal portion, the proximal-end portion, or the proximal length of the ultrasound probe is not a terminal portion or terminal length of the ultrasound probe.

With respect to "distal," a "distal portion" or a "distal-end portion" of, for example, an ultrasound probe disclosed herein includes a portion of the ultrasound probe intended to be near or in a patient when the ultrasound probe is used on the patient. Likewise, a "distal length" of, for example, the ultrasound probe includes a length of the ultrasound probe intended to be near or in the patient when the ultrasound probe is used on the patient. A "distal end" of, for example, the ultrasound probe includes an end of the ultrasound probe intended to be near or in the patient when the ultrasound probe is used on the patient. The distal portion, the distal-end portion, or the distal length of the ultrasound probe can include the distal end of the ultrasound probe; however, the distal portion, the distal-end portion, or the distal length of the ultrasound probe need not include the distal end of the ultrasound probe. That is, unless context suggests otherwise, the distal portion, the distal-end portion, or the distal length of the ultrasound probe is not a terminal portion or terminal length of the ultrasound probe.

Unless defined otherwise, all technical and scientific terms used herein have the same meaning as commonly understood by those of ordinary skill in the art.

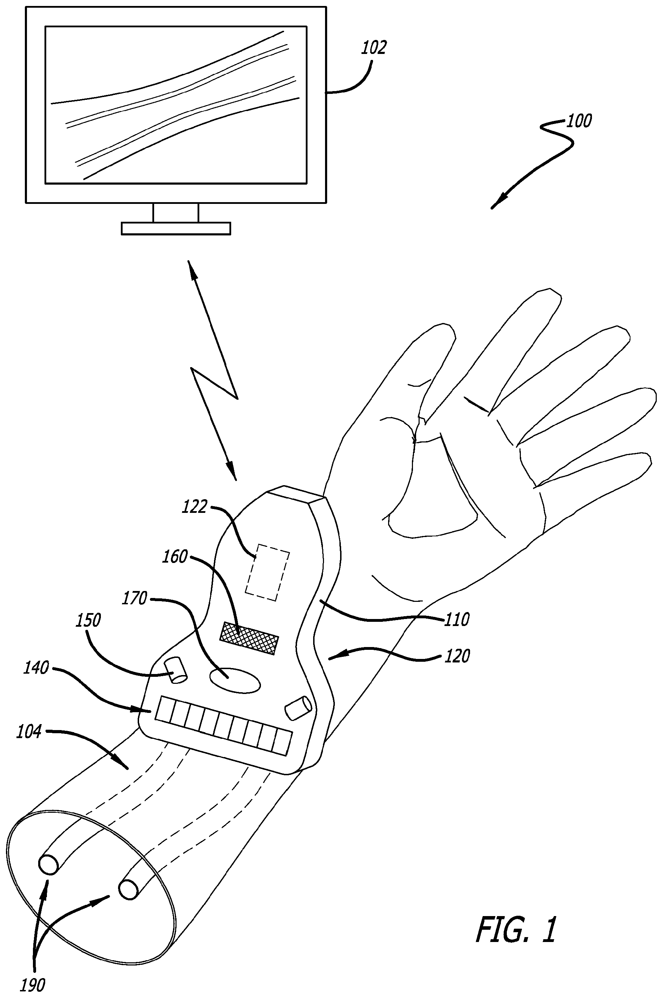

FIG. 1 illustrates a perspective view of a medical device system 100 including one or more blood vessel correlation tools 120, in accordance with some embodiments. In some embodiments, the medical device system 100 includes an ultrasound probe 110 configured to detect one or more blood vessels 190 in a target area 104. In some embodiments, the ultrasound probe 110 may be in communication with a display screen 102 configured to display a depiction of the one or more detected blood vessels 190 in the target area 104. In some embodiments, the ultrasound probe 110 may be in wireless communication with the display screen 102. Exemplary wireless communication modalities can include WiFi, Bluetooth, Near Field Communications (NFC), cellular Global System for Mobile Communication ("GSM"), electromagnetic (EM), radio frequency (RF), combinations thereof, or the like.

In some embodiments, the ultrasound probe 110 may include one or more blood vessel correlation tools 120 thereon. The one or more blood vessel correlation tools 120 may be in communication with the ultrasound probe 110. The one or more blood vessel correlation tools 120 may be configured to receive the one or more detected blood vessel data from the ultrasound probe 110. The one or more blood vessel correlation tools 120 may be configured to correlate the detected blood vessel data with a location of the one or more blood vessels within the target area 104. The one or more blood vessel correlation tools 120 may be configured to generate a local output that can be detected by a user, the local output indicating one or more detected parameters of the detected blood vessels 190. In some embodiments, the ultrasound probe 110 may be configured to detect the one or more blood vessels 190 by ultrasound waves. In some embodiments, the ultrasound probe 110 may be configured to detect the one or more blood vessels 190 by near infrared reflection differentiation using a near infrared or infrared emitter and detector.

The one or more blood vessel correlation tools 120 may include any one or more of the following: a single light or a light array 140, one or more visible light projectors 150, a haptic feedback system 160, and an auditory device 170. The one or more blood vessel correlation tools 120 may be in communication with a console 122 configured to provide communication between the ultrasound probe 110 and the one or more blood vessel correlation tools 120. In some embodiments, the local output may include a local visual output, a local haptic output, a local auditory output, or a combination thereof. The local output may indicate one or more parameters including blood vessel type (e.g., vein or artery), blood vessel size (e.g., diameter, cross sectional area, or the like), blood vessel location, blood vessel depth, or appropriate vascular access device that may access the detected blood vessels 190. Advantageously, the local output of the medical device system 100 allows the user to focus their entire attention on the target area 104 and receive data on the various parameters of the detected one or more blood vessels 190 near the target area 104 or within the target area 104 instead of receiving data the various parameters of the detected one or more blood vessels on the display screen 102 away from the target area 104.

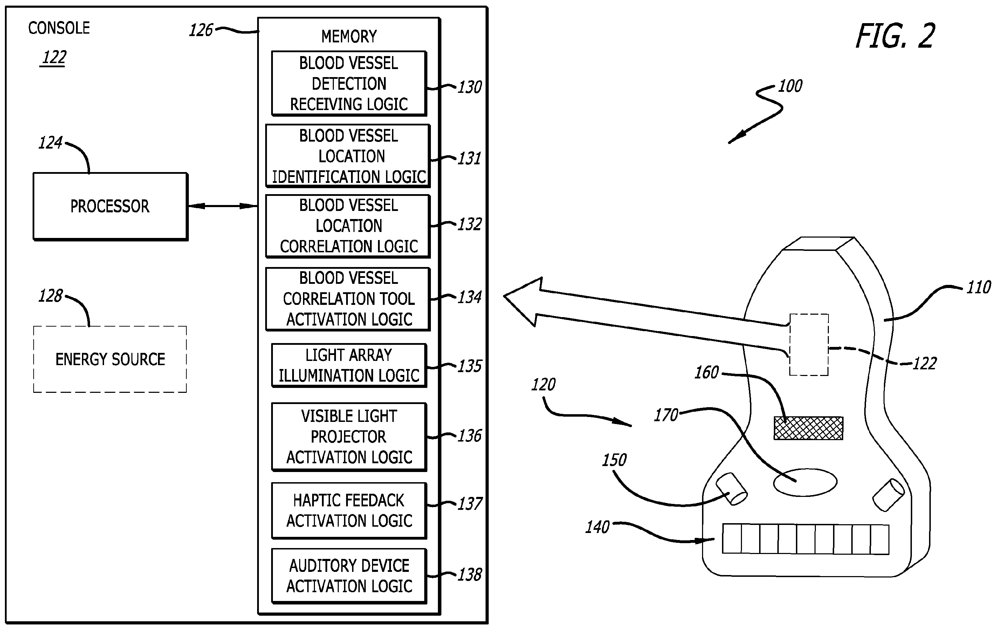

FIG. 2 illustrates a block diagram of some components of the medical device system 100 including the ultrasound probe 110 and the console 122, in accordance with some embodiments. In some embodiments, the ultrasound probe 110 of the medical device system 100 includes the console 122 therein configured to control the one or more vessel correlation tools 120. In some embodiments, the console 122 includes one or more processors 124, non-transitory computer readable medium ("memory") 126, an energy source 128 and a plurality of logic modules. The console 122 is in communication with the ultrasound probe 110 and each of the one or more blood vessel correlation tools 120. The plurality of logic modules may be configured to include one or more of: a blood vessel detection receiving logic 130, a blood vessel location identification logic 131, a blood vessel location correlation logic 132, and a blood vessel correlation tool activation logic 134. In some embodiments, the blood vessel detection receiving logic 130 may be configured to receive the blood vessel detection data from the ultrasound probe 110.

In some embodiments, the blood vessel detection data may include the detected ultrasound wave data or the detected near-infrared reflection differentiation data. In some embodiments, the blood vessel location identification logic 131 may be configured to identify the location of the one or more blood vessels 190 from the detected blood vessel data. In some embodiments, the blood vessel location identification logic 131 may be configured to identify the location of the one or more blood vessels 190 within one or more captured images from the ultrasound probe 110. In some embodiments, the blood vessel location identification logic 131 may be configured to identify the location of the one or more blood vessels 190 within the target area 104. In some embodiments, the blood vessel location correlation logic 132 may be to correlate the location of the one or more blood vessels 190 within the target area 104 in generating the local output. In some embodiments, the blood vessel correlation tool activation logic 134 may be configured to activate the one or more blood vessel correlation tools 120 to indicate to a user, the location of the one or more blood vessels 190 within the target area 104. In some embodiments, the blood vessel correlation tool activation logic 134 may be configured to simultaneously activate two or more of the blood vessel correlation tools 120. In some embodiments, activating the one or more blood vessel correlation tools 120 generates the local output configured to be detected by the user, the local output identifying one or more parameters of the detected blood vessels 190 in the target area 104. In some embodiments, simultaneously activating two or more blood vessel correlation tools generates two or more distinct local outputs configured to identify, to the user, two or more parameters of the detected blood vessels 190. In some embodiments, the two or more local outputs may be configured to identify the same two or more parameters of the detected blood vessels 190 or different parameters of the detected blood vessels 190.

In some embodiments, the blood vessel correlation tool activation logic 134 may include sub-logic specific to the blood vessel correlation tool used. For example, the sub-logic may be configured to include a light array illumination logic 135, a visible light projection activation logic 136, a haptic feedback activation logic 137, and an auditory device activation logic 138. In some embodiments, the light array illumination logic 135 may be configured to illuminate one or more of the lights within the light array 140. In some embodiments, the light array illumination logic 135 may be configured to illuminate the one or more lights within the light array 140 different colors to identify different blood vessel types (e.g., artery versus vein) or differing blood vessel sizes (e.g., cross sectional area), indicating an appropriate vascular access device for the detected blood vessel 190. In some embodiments, the light array illumination logic 135 may be configured to vary the brightness of the one or more lights within the light array 140 to indicate different blood vessel depth, different blood vessel size or the like.

In some embodiments, the visible light projection activation logic 136 may be configured to project a depiction of the detected blood vessels 190 within the target area 104, including on a skin surface. In some embodiments, the visible light projection activation logic 136 may be configured to vary the projected depiction of the detected blood vessels 190 within the target area 104 by varying the shape, the location, the color, or the brightness of the depiction to indicate different blood vessel depth, different blood vessel size, different blood vessel type, different blood vessel location or different vascular access devices that may access the detected blood vessels 190.

In some embodiments, the haptic feedback activation logic 137 may be configured to provide haptic feedback to the user through vibration of a portion of the ultrasound probe 110 including the haptic feedback system 160. In some embodiments, the haptic feedback activation logic 137 may be configured to vary a vibration pulse by vibration pulse frequency and/or vibration pulse intensity to indicate different blood vessel depth, different blood vessel size, different blood vessel type, different blood vessel location or different vascular access devices that may access the detected blood vessels 190.

In some embodiments, the auditory device activation logic 138 may be configured to provide one or more auditory signals from the auditory device 170. In some embodiments, the auditory signals may include one or more sonic tones, instructions of the location of the blood vessels 190 within the target area 104, or other auditory signals. In some embodiments, the auditory device activation logic 138 may be configured to vary the one or more auditory signals from the auditory device 170 by varying sonic tone frequency, sonic tone pitch, or sonic tone intensity to indicate to the user different blood vessel depth, different blood vessel size, different blood vessel type, different blood vessel location or different vascular access devices that may access the detected blood vessels 190.

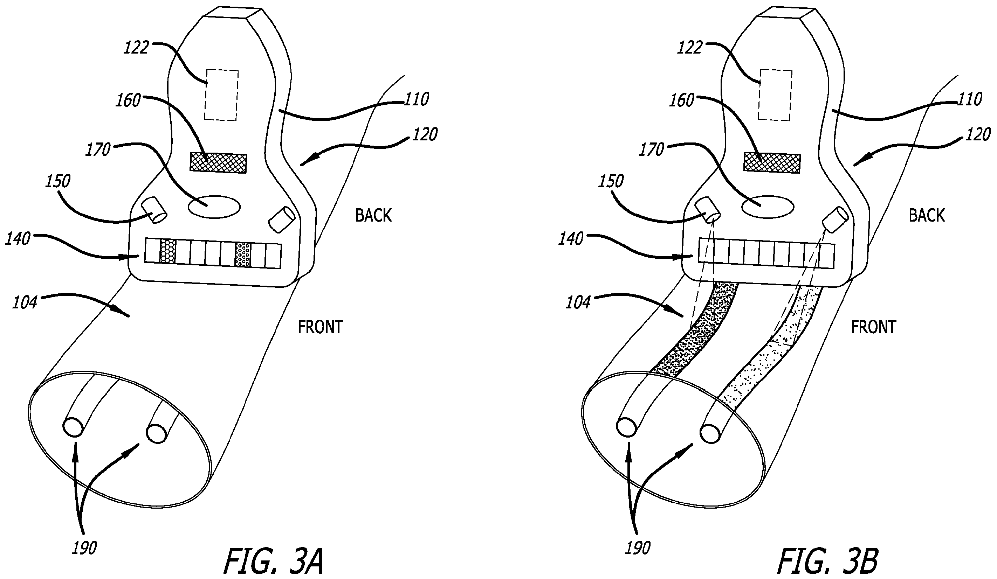

FIG. 3A illustrates a perspective view of the ultrasound probe 110 including the light array 140 coupled thereto, in accordance with some embodiments. In some embodiments, the single light or the light array 140 may be coupled to the ultrasound probe 110. In some embodiments, the light array 140 may be detachably coupled to the probe 110. In some embodiments, the light array 140 may be coupled to a front surface or a back surface, orientating the ultrasound probe 110 for proper use. The light array 140 includes a plurality of individual lights arranged in an array. In some embodiments, the individual lights may include light-emitting diodes ("LEDs"). The light array 140 may be configured to indicate the location of the one or more blood vessels 190 within the target area 104. The individual lights or multiple sequential lights in the light array 140 may be illuminated, as the local output, to indicate the location of the one or more blood vessels 190 directly below the light array 140. In some embodiments, the illuminated lights in the light array 140 may be configured to indicate to the user, other parameters of the one or more detected blood vessels 190. For example, the lights within the light array 140 may be illuminated different colors and varied brightness to correspond to different blood vessel types (e.g., artery versus vein), different blood vessel sizes, different blood vessel depth or different vascular access devices that may be configured to access the one or more blood vessels 190.

FIG. 3B illustrates a perspective view of the ultrasound probe 110 including the one or more visible light projectors 150 coupled thereto, in accordance with some embodiments. In some embodiments, the one or more visible light projectors 150 may be coupled to the ultrasound probe 110 or embedded within the ultrasound probe 110. In some embodiments, the one or more visible light projectors 150 may be configured to display various visible light projections on the target area 104 including on a skin surface within the target area 104. In some embodiments, each visible light projector 150 may be configured to project one visible light projection within the target area or the visible light projectors 150 may be configured to combine to project one visible light projection within the target area 104. In some embodiments, the one or more visible light projectors 150 may include one or more lasers. In some embodiments, the visible light projections may include one or more blood vessel visualization depictions. In some embodiments, the visible light projections may include various shapes or text. In some embodiments, the one or more visible light projections may be distinct, corresponding to different parameters of the one or more blood vessels. In some embodiments, the one or more visible light projections may be configured to display projections of different colors or of different brightness. In some embodiments, the different colors or different brightness may correspond to different blood vessel types, different blood vessel sizes, different blood vessel depth or different vascular access devices that may be configured to access the one or more blood vessels 190.

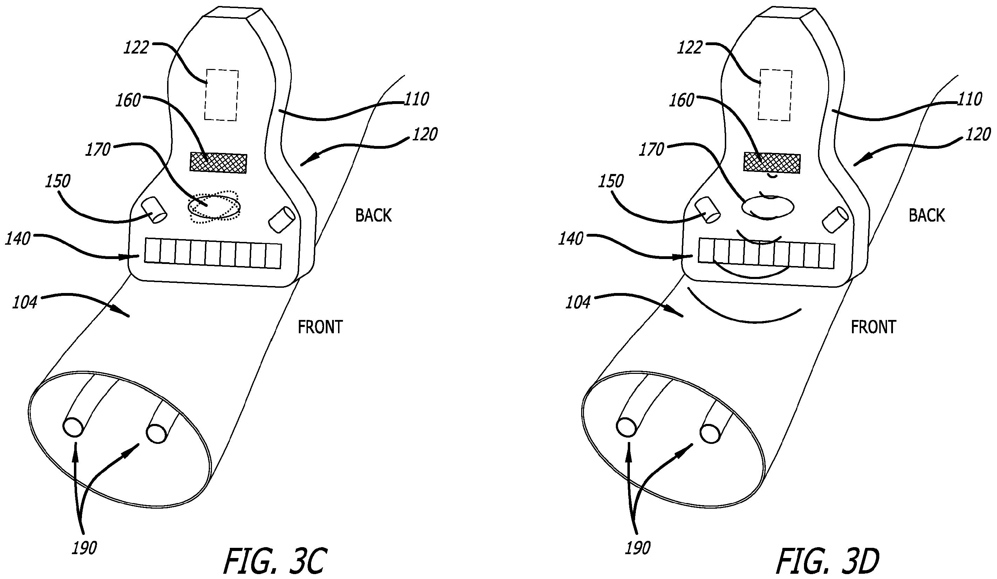

FIG. 3C illustrates a perspective view of the ultrasound probe 110 including the haptic feedback system 160 in accordance with some embodiments. In some embodiments, the one or more blood vessel correlation tools 120 may include the haptic feedback system 160 configured to indicate the location of one or more blood vessels 190 to the user. In some embodiments, the haptic feedback system 160 may be configured to indicate other parameters of the detected blood vessels including blood vessel depth, blood vessel size, blood vessel type, or different vascular access devices that may be configured to access the one or more blood vessels 190. In some embodiments, the haptic feedback system 160 may be configured to indicate the parameters of the one or more blood vessels 190 through varied frequency or varied magnitude of pulses or vibrations.

FIG. 3D illustrates a perspective view of the ultrasound probe 110 including the auditory device 170, in accordance with some embodiments. In some embodiments, the one or more blood vessel correlation tools 120 may include the auditory device 170 coupled to the ultrasound probe 110. In some embodiments, the auditory device 170 may include a speaker in communication with the console 122. In some embodiments, the auditory device 170 may be integrated into the ultrasound probe 110. The auditory device 170 may be configured to identify the one or more blood vessels 190 to the user, using various sounds (e.g., auditory messages or the like) or sonic tones that may be varied in frequency or pitch. In some embodiments, the auditory device 170 may be configured to indicate other parameters of the blood vessels 190 to the user, including blood vessel depth, blood vessel size, blood vessel type or different vascular access devices that may be configured to access the one or more blood vessels 190.

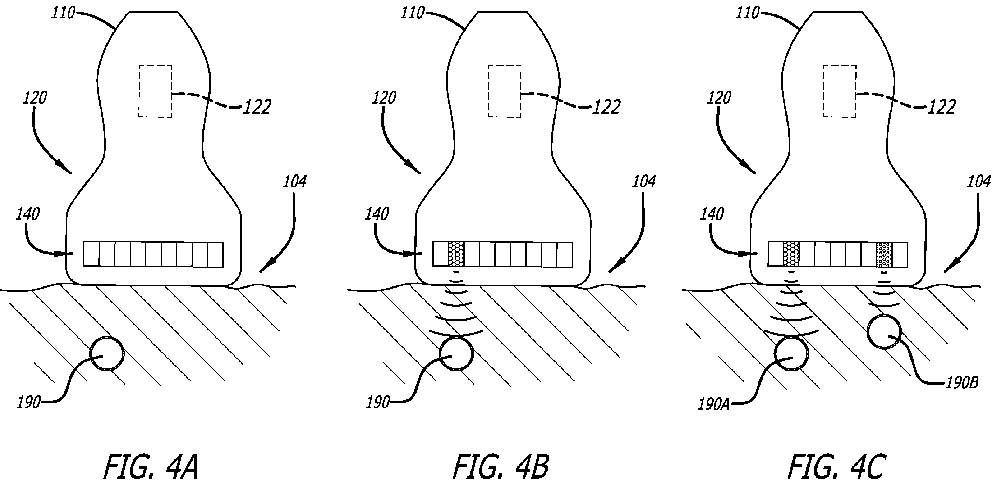

FIGS. 4A-4C illustrate a cross sectional view of detecting one or more blood vessels 190 within the target area 104 and generating a local output configured to be detected by a user, in accordance with some embodiments. As illustrated in FIG. 4A, the ultrasound probe 110 may be used to detect one or more blood vessels 190 within the target area 104. The ultrasound probe 110 may detect the one or more blood vessels 190 by ultrasound. The console 122 may correlate one of the parameters of the detected blood vessel 190 with a local output, the local output being transmitted to the one of the blood vessel correlation tools 120. As illustrated in FIG. 4B, the blood vessel correlation tool 120 illustrated includes the light array 140. The console 122 may illuminate one of the lights in the light array 140 a first color, indicating the location of the detected blood vessel 190 within the target area 104. The console 122 may illuminate one of the lights in a particular pattern to indicate the depth of the detected blood vessel 190. As illustrated in FIG. 4C, when the ultrasound probe 110 detects the first blood vessel 190A and the second blood vessel 190B within the target area 104, the console 122 may illuminate one of the lights in the light array 140 a first color and another light in the light array 140 a second color, both colors indicating the location of the first and second blood vessels 190A-190B within the target area 104. The console 122 may illuminate each light in the light array 140 a different pattern, to indicate the depth of the first detected blood vessel 190A versus the depth of the second detected blood vessel 190B.

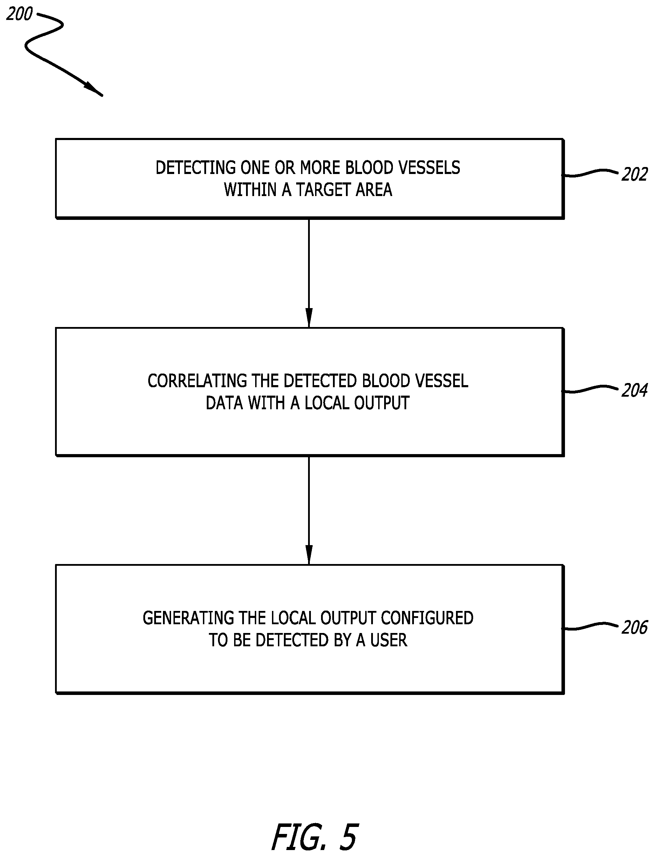

FIG. 5 illustrates a flow chart of the exemplary method 200 of detecting one or more blood vessels 190 within a target area 104 and correlating the detected blood vessels 190 with a local output detected by a user, in accordance with some embodiments. In some embodiments, the method 200 includes detecting one or more blood vessels 190 within a target area 104 (block 202). In some embodiments, detecting the one or more blood vessels 190 within the target area 104 includes detecting using the ultrasound probe 110 configured to detect using ultrasound or near-infrared reflection differentiation.

The method 200 further includes correlating detected blood vessel data with a local output corresponding to one or more parameters of the detected blood vessels (block 204). In some embodiments, the local output may correspond to one or more parameters of the detected blood vessels 190. In some embodiments, the one or more parameters may include blood vessels type, blood vessel size, blood vessel depth, blood vessel location or vascular access devices configured to access the detected blood vessels 190. In some embodiments, correlating the detected blood vessel data includes the console 122 receiving the detected blood vessel data from the ultrasound probe 110.

The method 200 includes generating the local output configured to be detected by a user using one or more blood vessel correlation tools 120 (block 206). In some embodiments, the one or more blood vessel correlation tools 120 include a single light or a light array 140, one or more visible light projectors 150, a haptic feedback system 160, and an auditory device 170. In some embodiments, generating the local output includes illuminating one or more lights in the light array 140. In some embodiments, illuminating one or more lights in the light array 140 includes illuminating the one or more lights in the light array 140 different colors, different patterns, or of a varying brightness, wherein each of the different colors, patterns, or brightness corresponds to one parameter of the one or more parameters.

In some embodiments, generating the local output configured to be detected by the user using one or more blood vessel correlation tools 120 includes the one or more visible light projectors 150 projecting a blood vessel visualization depiction on the target area 104. In some embodiments, the blood vessel visualization depiction may include different shapes or different colors, wherein each of the different shapes or different colors corresponds to one parameter of the one or more parameters.

In some embodiments, generating the local output configured to be detected by the user using one or more blood vessel correlation tools 120 includes the haptic feedback system 160 vibrating a portion of the ultrasound probe 110 in various patterns, various frequencies, or various magnitudes, wherein each of the various patterns, frequencies, or magnitudes corresponds to one parameter of the one or more parameters. In some embodiments, generating the local output configured to be detected by the user using one or more blood vessel correlation tools 120 includes the auditory device 170 emitting one or more sonic tones having various frequencies, pitches, or patterns, wherein each of the various frequencies, pitches, or patterns correspond to one parameter of the one or more parameters.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of protection as defined by the appended claims.

## Claims

1. A medical device system configured to detect one or more blood vessels within a target area, comprising:
an ultrasound probe (110) configured to detect the one or more blood vessels, the ultrasound probe in communication with a console (122); and one or more blood vessel correlation tools (120) of the ultrasound probe, the one or more blood vessel correlation tools in communication with the console and configured to generate a local output detectable by a user, the local output corresponding to one or more parameters of the one or more blood vessels selected from at least blood vessel type, blood vessel depth, and blood vessel size, and the one or more blood vessel correlation tools including:
a light array (140) of light-emitting diodes ("LEDs") configured to indicate the one or more parameters of the one or more blood vessels by illuminating the LEDs different colors, and/or varying brightness of the LEDs, wherein illuminating one of the LEDs indicates a location of a detected blood vessel within the target area with respect to the ultrasound probe (110).

2. The medical device system according to claim 1, wherein the ultrasound probe (110) further comprises a near infrared emitter and detector, and is configured to detect the one or more blood vessels within the target area using ultrasound waves or near-infrared reflection differentiation by way of the near infrared emitter and detector of the ultrasound probe.

3. The medical device system according to either claim 1 or claim 2, wherein the console (122) includes one or more processors (124), an energy source (128), a non-transitory computer readable medium (126), and a plurality of logic modules (131, 132, 133, 134, 135, 136, 137, 138).

4. The medical device system according to claim 3, wherein the plurality of logic modules (131, 132, 133, 134, 135, 136, 137, 138), when activated by the one or more processors, are configured to perform operations including:
receiving detected blood vessel data from the ultrasound probe (110);
correlating the detected blood vessel data with the local output corresponding to the one or more parameters of the one or more blood vessels; and generating the local output that is detectable by the user.

5. The medical device system according to claim 4, wherein the one or more parameters further include blood vessel location within the target area, blood vessel size, blood vessel depth or vascular access devices that are configured to access the one or more blood vessels.

6. The medical device system according to claim 4, wherein the local output includes visible illumination of one or more LEDs in the light array (140), optionally
wherein the one or more LEDs in the light array (140) are further configured to indicate the one or more parameters of the one or more blood vessels by illuminating the one or more LEDs different patterns.

7. The medical device system according to claim 4, wherein the one or more blood vessel correlation tools further comprises one or more visible light projectors (150), wherein the local output includes the one or more visible light projections by the one or more visible light projectors (150) within the target area, optionally
wherein the one or more visible light projectors (150) are lasers, or wherein the one or more visible light projectors (150) are further configured to project text to indicate the one or more parameters of the one or more blood vessels.

8. The medical device system according to claim 4, wherein the one or more blood vessel correlation tools further comprises an auditory device (170), wherein the local output includes the auditory signal from the auditory device (170), optionally
wherein the auditory signal further includes messages configured to indicate the one or more parameters of the one or more blood vessels.

9. The medical device system according to claim 4, wherein the one or more blood vessel correlation tools further comprises a haptic feedback system (160), wherein the local output includes the vibrations of the portion of the ultrasound probe through the haptic feedback system (160).

10. The medical device system according to claim 9, wherein the haptic feedback system (160) is further configured to indicate the one or more parameters of the one or more blood vessels by varying a vibration pulse by vibration pulse frequency and/or pulse intensity.

11. A method of detecting one or more blood vessels within a target area and generating a local output detected by a user, comprising:
detecting the one or more blood vessels within the target area using an ultrasound probe (110) in communication with a console (122);
correlating detected blood vessel data with a local output, the local output corresponding to one or more parameters of the one or more blood vessels selected from at least blood vessel type, blood vessel depth, and blood vessel size; and
generating the local output configured to be detected by a user using one or more blood vessel correlation tools (120) of the ultrasound probe (110), the one or more blood vessel correlation tools including:
a light array (140) of light-emitting diodes ("LEDs") configured to indicate the one or more parameters of the one or more blood vessels by illuminating the LEDs different colors, and/or varying brightness of the LEDs, wherein illuminating one of the LEDs indicates a location of a detected blood vessel within the target area with respect to the ultrasound probe.

12. The method according to claim 11, wherein detecting the one or more blood vessels in the target area using the ultrasound probe (110) includes detecting the one or more blood vessels by ultrasound waves or near-infrared reflection differentiation by way of a near infrared emitter and detector of the ultrasound probe.

13. The method according to claim 12, wherein the one or more parameters include blood vessel location , blood vessel type, blood vessel size, and vascular access devices configured to access the one or more blood vessels.

14. The method according to claim 13, wherein the local output includes visible illumination of the one or more LEDs in the light array (140), the one or more LEDs in the light array further configured to indicate the one or more parameters of the one or more blood vessels by varying illuminating the one or more LEDs different patterns.

15. The method according to claim 13, wherein the one or more blood vessel correlation tools (120) further comprises one or more visible light projectors (150), wherein the local output includes the one or more visible light projections by the one or more visible light projectors (150) within the target area, the one or more visible light projectors (150) being lasers, or
wherein the one or more blood vessel correlation tools (120) further comprises a haptic feedback system (160), wherein the local output includes the vibrations of the portion of the ultrasound probe through the haptic feedback system (160), the haptic feedback system (160) further configured to indicate the one or more parameters of the one or more blood vessels by varying a vibration pulse by vibration pulse frequency and/or pulse intensity, or
wherein the one or more blood vessel correlation tools further comprises an auditory device (170), wherein the local output includes the auditory signal from the auditory device (170), the auditory signal further including messages configured to indicate the one or more parameters of the one or more blood vessels.

## Patentansprüche

1. Medizinisches Vorrichtungssystem, das ausgebildet ist, eines oder mehrerer Blutgefäße innerhalb eines Zielbereichs zu erkennen, umfassend:
eine Ultraschallsonde (110), die das eine oder mehrere Blutgefäße erkennt, wobei die Ultraschallsonde mit einer Konsole (122) in Verbindung steht; und
ein oder mehrere Blutgefäßkorrelationswerkzeuge (120) der Ultraschallsonde, wobei das eine oder die mehreren Blutgefäßkorrelationswerkzeuge mit der Konsole in Verbindung stehen und ausgebildet sind, um eine lokale Ausgabe zu erzeugen,
die für einen Benutzer wahrnehmbar ist, wobei die lokale Ausgabe einem oder mehreren Parametern des einen oder der mehreren Blutgefäße entspricht, die aus mindestens dem Blutgefäßtyp, der Blutgefäßtiefe und der Blutgefäßgröße ausgewählt sind, und wobei das eine oder die mehreren Blutgefäßkorrelationswerkzeuge Folgendes einschließen:
ein Lichtarray (140) aus Leuchtdioden ("LEDs"), das so ausgebildet ist, dass es den einen oder die mehreren Parameter des einen oder der mehreren Blutgefäße durch Leuchten der LEDs in unterschiedlichen Farben und/oder durch Variieren der Helligkeit der LEDs anzeigt, wobei das Leuchten einer der LEDs einen Standort eines erkannten Blutgefäßes innerhalb des Zielbereichs in Bezug auf die Ultraschallsonde (110) anzeigt.

2. Medizinisches Vorrichtungssystem nach Anspruch 1, wobei die Ultraschallsonde (110) weiter einen Nahinfrarot-Emitter und -Detektor umfasst und so ausgebildet ist, dass sie das eine oder die mehrere Blutgefäße innerhalb des Zielbereichs mithilfe von Ultraschallwellen oder durch Nahinfrarot-Reflexionsdifferenzierung mittels des Nahinfrarot-Emitters und -Detektors der Ultraschallsonde erkennt.

3. Medizinisches Vorrichtungssytem nach Anspruch 1 oder Anspruch 2, wobei die Konsole (122) einen oder mehrere Prozessoren (124), eine Energiequelle (128), ein nichtflüchtiges, computerlesbares Medium (126) und eine Vielzahl von Logikmodulen (131, 132, 133, 134, 135, 136, 137, 138) einschließt.

4. Medizinisches Vorrichtungssystem nach Anspruch 3, wobei die Vielzahl von Logikmodulen (131, 132, 133, 134, 135, 136, 137, 138) bei Aktivierung durch den einen oder die mehrere Prozessoren so ausgebildet sind, dass sie unter anderem folgende Vorgänge ausführen:
Empfangen von erkannten Blutgefäßdaten von der Ultraschallsonde (110);
Korrelieren der erkannten Blutgefäßdaten mit dem lokalen Ausgangssignal, das den einen oder mehreren Parametern des einen oder der mehreren Blutgefäße entspricht; und Erzeugen des lokalen Ausgangssignals, das für den Benutzer wahrnehmbar ist.

5. Medizinisches Vorrichtungssystem nach Anspruch 4, wobei der eine oder die mehreren Parameter weiter den Standort des Blutgefäßes innerhalb des Zielbereichs, die Größe des Blutgefäßes, die Tiefe des Blutgefäßes oder Gefäßzugangsvorrichtungen einschließen, die ausgebildet sind, um den Zugang zu dem einen oder den mehreren Blutgefäßen zu ermöglichen.

6. Medizinisches Vorrichtungssystem nach Anspruch 4, wobei die lokale Ausgabe das Leuchten einer oder mehrerer LEDs in dem Lichtarray (140) einschließt, optional
wobei die eine oder mehreren LEDs in dem Lichtarray (140) weiter so ausgebildet sind, dass sie die einen oder mehreren Parameter der einen oder mehreren Blutgefäße anzeigen, indem die eine oder mehreren LEDs unterschiedliche Muster leuchten.

7. Medizinisches Vorrichtungssystem nach Anspruch 4, wobei das eine oder die mehreren Blutgefäßkorrelationswerkzeuge weiter einen oder mehrere sichtbare Lichtprojektoren (150) umfasst, wobei die lokale Ausgabe die eine oder die mehreren sichtbaren Lichtprojektionen durch den einen oder die mehreren sichtbaren Lichtprojektoren (150) innerhalb des Zielbereichs einschließt, optional
wobei die einen oder mehreren sichtbaren Lichtprojektoren (150) Laser sind oder wobei die einen oder mehreren sichtbaren Lichtprojektoren (150) weiter so ausgebildet sind, dass sie Text projizieren, um die einen oder mehreren Parameter der einen oder mehreren Blutgefäße anzuzeigen.

8. Medizinisches Vorrichtungssystem nach Anspruch 4, wobei das eine oder die mehreren Blutgefäßkorrelationswerkzeuge weiter eine akustische Vorrichtung (170) umfassen, wobei die lokale Ausgabe das akustische Signal der akustischen Vorrichtung (170) einschließt, optional
wobei das akustische Signal weiter Nachrichten einschließt, die so ausgebildet sind, dass sie den einen oder die mehreren Parameter des einen oder der mehreren Blutgefäße anzeigen.

9. Medizinisches Vorrichtungssystem nach Anspruch 4, wobei das eine oder die mehreren Blutgefäßkorrelationswerkzeuge weiter ein haptisches Rückmeldesystem (160) umfassen, wobei die lokale Ausgabe die Vibrationen des Abschnitts der Ultraschallsonde über das haptische Rückmeldesystem (160) einschließt.

10. Medizinisches Vorrichtungssystem nach Anspruch 9, wobei das haptische Rückmeldesystem (160) weiter so ausgebildet ist, dass es den einen oder die mehreren Parameter des einen oder der mehreren Blutgefäße durch Variation eines Vibrationsimpulses hinsichtlich der Impulsfrequenz und/oder der Impulsintensität anzeigt.

11. Verfahren zum Erkennen eines oder mehrerer Blutgefäße innerhalb eines Zielbereichs und zum Erzeugen einer lokalen Ausgabe, die von einem Benutzer wahrgenommen wird, umfassend:
Erkennen eines oder mehrerer Blutgefäße innerhalb des Zielbereichs unter Verwendung einer Ultraschallsonde (110), die mit einer Konsole (122) in Verbindung steht;
Korrelieren der erkannten Blutgefäßdaten mit einer lokalen Ausgabe, wobei die lokale Ausgabe einem oder mehreren Parametern des einen oder der mehreren Blutgefäße entspricht, die aus mindestens Blutgefäßtyp, Blutgefäßtiefe und Blutgefäßgröße ausgewählt sind; und
Erzeugen der lokalen Ausgabe, die ausgebildet ist, damit sie von einem Benutzer mithilfe eines oder mehrerer Blutgefäßkorrelationswerkzeuge (120) der Ultraschallsonde (110) erkannt werden kann, wobei das eine oder die mehreren Blutgefäßkorrelationswerkzeuge Folgendes einschließen:
ein Lichtarray (140) aus Leuchtdioden ("LEDs"), das so ausgebildet ist, dass es den einen oder die mehreren Parameter des einen oder der mehreren Blutgefäße durch Leuchten der LEDs in unterschiedlichen Farben und/oder durch Variieren der Helligkeit der LEDs anzeigt, wobei das Leuchten einer der LEDs einen Standort eines erkannten Blutgefäßes innerhalb des Zielbereichs in Bezug auf die Ultraschallsonde anzeigt.

12. Verfahren nach Anspruch 11, wobei das Erkennen des einen oder der mehreren Blutgefäße im Zielbereich unter Verwendung der Ultraschallsonde (110) das Erkennen des einen oder der mehreren Blutgefäße mittels Ultraschallwellen oder durch Unterscheidung anhand der Reflexion im nahen Infrarotbereich unter Einsatz eines nahinfraroten Emitters und Detektors der Ultraschallsonde einschließt.

13. Verfahren nach Anspruch 12, wobei die einen oder die mehreren Parameter den Standort des Blutgefäßes, den Blutgefäßtyp, die Blutgefäßgröße und Gefäßzugangsvorrichtungen einschließen, die dafür ausgebildet sind, Zugang zu dem einen oder den mehreren Blutgefäßen zu erhalten.

14. Verfahren nach Anspruch 13, wobei die lokale Ausgabe die sichtbare Beleuchtung der einen oder mehreren LEDs in dem Lichtarray (140) einschließt, wobei die eine oder mehreren LEDs in dem Lichtarray weiter so ausgebildet sind, dass sie die einen oder mehreren Parameter der einen oder mehreren Blutgefäße durch unterschiedliche Beleuchtungsmuster der einen oder mehreren LEDs anzeigen.

15. Verfahren nach Anspruch 13, wobei das eine oder die mehreren Blutgefäßkorrelationswerkzeuge (120) weiter einen oder mehrere sichtbare Lichtprojektoren (150) umfasst, wobei die lokale Ausgabe die eine oder die mehreren sichtbaren Lichtprojektionen durch den einen oder die mehreren sichtbaren Lichtprojektoren (150) innerhalb des Zielbereichs einschließt, wobei der eine oder die mehreren sichtbaren Lichtprojektoren (150) Laser sind, oder
wobei das eine oder die mehreren Blutgefäßkorrelationswerkzeuge (120) weiter ein haptisches Rückmeldesystem (160) umfasst, wobei die lokale Ausgabe die Vibrationen des Abschnitts der Ultraschallsonde über das haptische Rückmeldesystem (160) umfasst, wobei das haptische Rückmeldesystem (160) weiter so ausgebildet ist, dass es den einen oder die mehreren Parameter des einen oder der mehreren Blutgefäße durch Variation eines Vibrationsimpulses hinsichtlich der Impulsfrequenz und/oder der Impulsintensität anzeigt, oder
wobei das eine oder die mehreren Blutgefäßkorrelationswerkzeuge weiter eine akustische Vorrichtung (170) umfassen, wobei die lokale Ausgabe das akustische Signal von der akustischen Vorrichtung (170) einschließt, und das akustische Signal weiter Nachrichten einschließt, die ausgebildet sind, um den einen oder die mehrere Parameter der einen oder der mehreren Blutgefäße anzuzeigen.

## Revendications

1. Système de dispositif médical configuré pour détecter un ou plusieurs vaisseaux sanguins dans une zone cible, comprenant :
une sonde à ultrasons (110) configurée pour détecter les un ou plusieurs vaisseaux sanguins, la sonde à ultrasons étant en communication avec une console (122) ; et
un ou plusieurs outils (120) de corrélation de vaisseau sanguin de la sonde à ultrasons, les un ou plusieurs outils de corrélation de vaisseau sanguin étant en communication avec la console et configurés pour générer une sortie locale détectable par un utilisateur, la sortie locale correspondant à un ou plusieurs paramètres des un ou plusieurs vaisseaux sanguins sélectionnés parmi au moins le type de vaisseau sanguin, la profondeur du vaisseau sanguin et la taille du vaisseau sanguin, et les un ou plusieurs outils de corrélation de vaisseau sanguin incluant :
un réseau lumineux (140) de diodes électroluminescentes (« DEL ») configuré pour indiquer les un ou plusieurs paramètres des un ou plusieurs vaisseaux sanguins en illuminant les DEL de couleurs différentes, et/ou en faisant varier la luminosité des DEL, dans lequel l'éclairage de l'une des DEL indique un emplacement d'un vaisseau sanguin détecté dans la zone cible par rapport à la sonde à ultrasons (110).

2. Système de dispositif médical selon la revendication 1, dans lequel la sonde à ultrasons (110) comprend en outre un émetteur et détecteur de proche infrarouge, et est configurée pour détecter les un ou plusieurs vaisseaux sanguins dans la zone cible à l'aide d'ondes ultrasonores ou d'une différenciation par réflexion dans le proche infrarouge par l'intermédiaire de l'émetteur et détecteur de proche infrarouge de la sonde à ultrasons.

3. Système de dispositif médical selon la revendication 1 ou revendication 2, dans lequel la console (122) inclut un ou plusieurs processeurs (124), une source d'énergie (128), un support (126) non transitoire lisible par ordinateur et une pluralité de modules logiques (131, 132, 133, 134, 135, 136, 137, 138).

4. Système de dispositif médical selon la revendication 3, dans lequel la pluralité de modules logiques (131, 132, 133, 134, 135, 136, 137, 138), lorsqu'ils sont activés par les un ou plusieurs processeurs, sont configurés pour effectuer des opérations incluant :
la réception de données de vaisseau sanguin détecté en provenance de la sonde à ultrasons (110) ;
la corrélation des données de vaisseau sanguin détecté avec la sortie locale correspondant aux un ou plusieurs paramètres des un ou plusieurs vaisseaux sanguins ; et la génération de la sortie locale qui est détectable par l'utilisateur.

5. Système de dispositif médical selon la revendication 4, dans lequel les un ou plusieurs paramètres incluent en outre l'emplacement du vaisseau sanguin dans la zone cible, la taille du vaisseau sanguin, la profondeur du vaisseau sanguin ou des dispositifs d'accès vasculaire qui sont configurés pour accéder aux un ou plusieurs vaisseaux sanguins.

6. Système de dispositif médical selon la revendication 4, dans lequel la sortie locale inclut un éclairage visible d'une ou de plusieurs DEL dans le réseau lumineux (140), facultativement
dans lequel les une ou plusieurs DEL dans le réseau lumineux (140) sont en outre configurées pour indiquer les un ou plusieurs paramètres des un ou plusieurs vaisseaux sanguins en éclairant les une ou plusieurs DEL selon des motifs différents.

7. Système de dispositif médical selon la revendication 4, dans lequel les un ou plusieurs outils de corrélation de vaisseau sanguin comprennent en outre un ou plusieurs projecteurs de lumière visible (150), dans lequel la sortie locale inclut les une ou plusieurs projections de lumière visible par les un ou plusieurs projecteurs de lumière visible (150) dans la zone cible, facultativement
dans lequel les un ou plusieurs projecteurs de lumière visible (150) sont des lasers, ou dans lequel les un ou plusieurs projecteurs de lumière visible (150) sont en outre configurés pour projeter du texte afin d'indiquer les un ou plusieurs paramètres des un ou plusieurs vaisseaux sanguins.

8. Système de dispositif médical selon la revendication 4, dans lequel les un ou plusieurs outils de corrélation de vaisseau sanguin comprennent en outre un dispositif auditif (170), dans lequel la sortie locale inclut le signal auditif provenant du dispositif auditif (170), facultativement
dans lequel le signal auditif inclut en outre des messages configurés pour indiquer les un ou plusieurs paramètres des un ou plusieurs vaisseaux sanguins.

9. Système de dispositif médical selon la revendication 4, dans lequel les un ou plusieurs outils de corrélation de vaisseau sanguin comprennent en outre un système de rétroaction haptique (160), dans lequel la sortie locale inclut les vibrations de la portion de la sonde à ultrasons à travers le système de rétroaction haptique (160).

10. Système de dispositif médical selon la revendication 9, dans lequel le système de rétroaction haptique (160) est en outre configuré pour indiquer les un ou plusieurs paramètres des un ou plusieurs vaisseaux sanguins en faisant varier une impulsion de vibration par fréquence d'impulsion et/ou intensité d'impulsion de vibration.

11. Procédé de détection d'un ou de plusieurs vaisseaux sanguins dans une zone cible et de génération d'une sortie locale détectée par un utilisateur, comprenant :
la détection des un ou plusieurs vaisseaux sanguins dans la zone cible à l'aide d'une sonde à ultrasons (110) en communication avec une console (122) ;
la corrélation de données de vaisseau sanguin détecté avec une sortie locale, la sortie locale correspondant à un ou plusieurs paramètres des un ou plusieurs vaisseaux sanguins sélectionnés parmi au moins le type de vaisseau sanguin, la profondeur du vaisseau sanguin et la taille du vaisseau sanguin ; et
la génération de la sortie locale configurée pour être détectée par un utilisateur à l'aide d'un ou de plusieurs outils (120) de corrélation de vaisseau sanguin de la sonde à ultrasons (110), les un ou plusieurs outils de corrélation de vaisseau sanguin incluant :
un réseau lumineux (140) de diodes électroluminescentes (« DEL ») configuré pour indiquer les un ou plusieurs paramètres des un ou plusieurs vaisseaux sanguins en éclairant les DEL de couleurs différentes, et/ou en faisant varier la luminosité des DEL, dans lequel l'éclairage de l'une des DEL indique un emplacement d'un vaisseau sanguin détecté dans la zone cible par rapport à la sonde à ultrasons.

12. Procédé selon la revendication 11, dans lequel la détection des un ou plusieurs vaisseaux sanguins dans la zone cible à l'aide de la sonde à ultrasons (110) inclut la détection des un ou plusieurs vaisseaux sanguins par ondes ultrasonores ou par différenciation par réflexion dans le proche infrarouge par l'intermédiaire d'un émetteur et détecteur de proche infrarouge de la sonde à ultrasons.

13. Procédé selon la revendication 12, dans lequel les un ou plusieurs paramètres incluent l'emplacement du vaisseau sanguin, le type de vaisseau sanguin, la taille du vaisseau sanguin et des dispositifs d'accès vasculaire configurés pour accéder aux un ou plusieurs vaisseaux sanguins.

14. Procédé selon la revendication 13, dans lequel la sortie locale inclut un éclairage visible des une ou plusieurs DEL dans le réseau lumineux (140), les une ou plusieurs DEL dans le réseau lumineux étant en outre configurées pour indiquer les un ou plusieurs paramètres des un ou plusieurs vaisseaux sanguins en faisant varier l'éclairage des une ou plusieurs DEL selon des motifs différents.

15. Procédé selon la revendication 13, dans lequel les un ou plusieurs outils (120) de corrélation de vaisseau sanguin comprennent en outre un ou plusieurs projecteurs de lumière visible (150), dans lequel la sortie locale inclut les une ou plusieurs projections de lumière visible par les un ou plusieurs projecteurs de lumière visible (150) dans la zone cible, les un ou plusieurs projecteurs de lumière visible (150) étant des lasers, ou
dans lequel les un ou plusieurs outils (120) de corrélation de vaisseau sanguin comprennent en outre un système de rétroaction haptique (160), dans lequel la sortie locale inclut les vibrations de la portion de la sonde à ultrasons à travers le système de rétroaction haptique (160), le système de rétroaction haptique (160) étant en outre configuré pour indiquer les un ou plusieurs paramètres des un ou plusieurs vaisseaux sanguins en faisant varier une impulsion de vibration par fréquence d'impulsion et/ou intensité d'impulsion de vibration, ou
dans lequel les un ou plusieurs outils de corrélation de vaisseau sanguin comprennent en outre un dispositif auditif (170), dans lequel la sortie locale inclut le signal auditif provenant du dispositif auditif (170), le signal auditif incluant en outre des messages configurés pour indiquer les un ou plusieurs paramètres des un ou plusieurs vaisseaux sanguins.
